# EUROPEAN PATENT SPECIFICATION

(11) **EP 2 968 758 B1**
(45) Date of publication and mention of the grant of the patent: **28.11.2018**
(21) Application number: 14779139.6
(22) Date of filing: 11.03.2014
(51) Int. Cl.: G16H 20/10, G16H 20/17

(54) **INFUSION ORDER AND DELIVERY CONSISTENCY**
INFUSIONSBESTELL- UND LIEFERKONSISTENZ
PRESCRIPTION DE PERFUSION ET COHÉRENCE D'ADMINISTRATION

(30) Priority: 13.03.2013 US 201313802663
(43) Date of publication of application: 20.01.2016
(73) Proprietor: CareFusion 303, Inc., San Diego, CA 92130 (US)
(72) Inventor: GARIBALDI, Federico, Encinitas, California 92024 (US); VANDERVEEN, Timothy W., Poway, California 92064 (US)
(74) Representative: Epping - Hermann - Fischer
(86) International application number: PCT/US2014/023685
(87) International publication number: WO 2014/164877

(56) References cited:
- WO-A2-2004/061745
- WO-A2-2004/061745
- US-A1- 2006 229 551
- US-A1- 2007 210 157
- US-A1- 2007 293 843
- US-A1- 2009 112 333
- US-A1- 2012 053 533

## Description

### CROSS-REFERENCE TO RELATED APPLICATIONS

The present application claims the benefit of priority under 35 U.S.C. §120 as a continuation in part from U.S. Patent Application Serial No. 09/860,865, entitled "Distributed Remote Asset and Medication Management Drug Delivery System," filed on May 18, 2001, which claims priority under 35 U.S.C. §119 from U.S. Provisional Application Serial No. 60/205,125, filed on May 18, 2000.

### BACKGROUND

### Field

The present disclosure generally relates to medical items, and more particularly to the verification of consistency between a medical item order and the medical item's use.

### Description of the Related Art

Intravenous (IV) fluid delivery systems are used to deliver fluids (*e.g.,* medicines, transfusions, *etc*.) to patients at controlled rates. Many individuals suffer from chronic health problems, the treatment of which requires regular, and sometimes extended, IV deliveries. Certain treatment regimens for diseases such as diabetes, asthma, epilepsy, cancer and even allergies, require the regular and sequenced infusion of precise amounts of intravenous medication for the patient's survival. Intravenous infusion of medications can take on many forms depending on the patient, treatment regimen, and choices of the clinician and institution. Treating chronic medical disorders often requires the administration of medication over a long period of time according to a treatment regimen specified by a medical professional, such as a physician.

In cases of patients admitted to a healthcare facility, one or more infusions to be administered to a patient are prescribed by the patient's physician. A pharmacy, generally located within the patient's hospital or healthcare facility, prepares the infusion medication or solution according to the physician's prescription. The pharmacist places the infusion solution in a bag, bottle, syringe, or other container and labels the container. The container is transported to the patient's location and a clinician such as a nurse or other clinician hangs the container from a rack. The nurse connects a tube between the container and an infusion pumping system and inserts a cannula at the end of the tube into the vessel of the patient.

A clinician either enters the programming data regarding the infusion into the pumping system manually, or the pumping system automatically loads the programming data from the label on the medication container or through a wired or wireless connection to another data source. The programming data may include, for example, the rate of infusion, the total volume to be infused, and in some cases, other delivery or operational parameters. The infusion pump is started and the infusion proceeds according to the programmed data. The clinician may perform a periodic monitoring of the patient and infusion according to the standard healthcare facility procedures. The pump will infuse the medication until it reaches the target volume to be infused, or until the pump empties the container. In certain instances, however, incorrect parameters may be entered for infusion of the medication, whether by the clinician or by the pumping system. The parameters may vary with the parameters indicated by an order for the medication.

If the clinician notes that the present medication container is about to be depleted, a replenishment container of the same medication could be ordered from the pharmacy, if so prescribed. Ideally, the new container would be delivered shortly prior to depletion of the present medication container, then upon actual depletion, the clinician would replace the emptied container with the new container, start the pump again, and the patient would continue to receive the prescribed medication with a very short interruption.

A clinician may, however, replenish a medication for infusion when an order to continue or otherwise replenish medication for the infusion does not exist. For example, if a patient is scheduled to be weaned off of infusion of a medication, a nurse or other clinician may replenish the medication for infusion when noticing that a current medication is depleted without checking the patient's infusion schedule. On the other hand, a clinician may inadvertently not provide a medication to a patient for infusion when an order to provide the medication to the patient exists. Even further, a clinician may enter programming data regarding the infusion into the pumping system that is not consistent with the parameters for infusing the medication in the medication order. Document WO2004061745 represents the closest prior art.

Document US20060229551discloses a medication management system.

### SUMMARY

The invention is defined by the appended claims. According to an embodiment of the present disclosure, a fluid medication delivery monitoring system is provided. The system includes a memory configured to receive first information indicating orders for fluid medications for patients, and second information indicating delivery status information for fluid medications for patients. The system also includes a processor configured to compare the orders for fluid medications for patients with the delivery status information for fluid medications for patients, and provide a notification when the comparison shows at least one of: one of the orders for fluid medication for a patient does not have a corresponding delivery status information for that order for fluid medication for the patient, or a delivery status information for fluid medication for a patient does not have a corresponding order for fluid medication for that patient.

According to an embodiment of the present disclosure, a method for monitoring delivery of a medication is provided. The method includes receiving first information indicating orders for fluid medications for patients, and second information indicating delivery status information for fluid medications for patients, and comparing the orders for fluid medications for patients with the delivery status information for fluid medications for patients. The method also includes providing a notification when the comparison shows at least one of: one of the orders for fluid medication for a patient does not have a corresponding delivery status information for that order for fluid medication for the patient, or a delivery status information for fluid medication for a patient does not have a corresponding order for fluid medication for that patient.

According to an embodiment of the present disclosure, a machine-readable storage medium includes machine-readable instructions for causing a processor to execute a method for monitoring delivery of a medication is provided. The method includes receiving first information indicating orders for fluid medications for patients, and second information indicating delivery status information for fluid medications for patients, and comparing the orders for fluid medications for patients with the delivery status information for fluid medications for patients. The method also includes providing a notification when the comparison shows at least one of: one of the orders for fluid medication for a patient does not have a corresponding delivery status information for that order for fluid medication for the patient, or a delivery status information for fluid medication for a patient does not have a corresponding order for fluid medication for that patient.

According to an embodiment of the present disclosure, a method for monitoring delivery of a medication is provided. The method includes receiving an indication that a patient associated with a medical device is unidentified, providing a notification indicating a location of the medical device in the institution, and sending, to a display associated with the medical device, a request to identify the patient associated with the medical device. The method also includes receiving an identification of the patient associated with the medical device, and comparing an order for fluid medication for the patient with the delivery status information for fluid medication for the patient. The method further includes providing a notification to the display associated with the medical device when the comparison shows at least one of: the order for fluid medication for the patient does not have a corresponding delivery status information for that order for fluid medication for the patient, or a delivery status information for fluid medication for the patient does not have a corresponding order for fluid medication for that patient.

It is understood that other configurations of the subject technology will become readily apparent to those skilled in the art from the following detailed description, wherein various configurations of the subject technology are shown and described by way of illustration. As will be realized, the subject technology is capable of other and different configurations and its several details are capable of modification in various other respects, all without departing from the scope of the subject technology. Accordingly, the drawings and detailed description are to be regarded as illustrative in nature and not as restrictive.

### BRIEF DESCRIPTION OF THE DRAWINGS

The accompanying drawings, which are included to provide further understanding and are incorporated in and constitute a part of this specification, illustrate disclosed embodiments and together with the description serve to explain the principles of the disclosed embodiments. In the drawings:
FIG. 1 illustrates an example architecture for monitoring delivery of a medication.
FIG. 2 is a block diagram illustrating an example monitoring system, hospital information system, and medical device from the architecture of FIG. 1 according to certain aspects of the disclosure.
FIG. 3 illustrates an example process for monitoring delivery of a medication using the example monitoring system of FIG. 2.
FIGS. 4A-4C are example illustrations associated with the example process of FIG. 3.
FIG. 5 is a block diagram illustrating an example computer system with which the clients and server of FIG. 2 can be implemented.

### DETAILED DESCRIPTION

In the following detailed description, numerous specific details are set forth to provide a full understanding of the present disclosure. It will be apparent, however, to one ordinarily skilled in the art that the embodiments of the present disclosure may be practiced without some of these specific details. In other instances, well-known structures and techniques have not been shown in detail so as not to obscure the disclosure.

In certain aspects, the disclosed fluid medication delivery monitoring system provides an alert or other notification when a medication (or "medication container") being infused to a patient is not consistent with an order for that medication. The inconsistency may be due to the order for medication not existing or the patient not receiving an ordered medication. In certain aspects, the inconsistency may be due to discrepancies between the medication delivery parameters in the order for the medication as compared to the medication delivery parameters of the medication currently being provided to the patient. As such, a clinician or other caregiver is alerted by the disclosed fluid medication delivery monitoring system. The alert can include a graphical display of a location of the infusion device within an institution whose current medication delivery is not consistent with an order of the medication. The alert can also include a notification sent to a device of a responsible caregiver, such as a mobile device, tablet computer, or desktop device. The alert can also include a notification sent to another device for further clinical decision making activities.

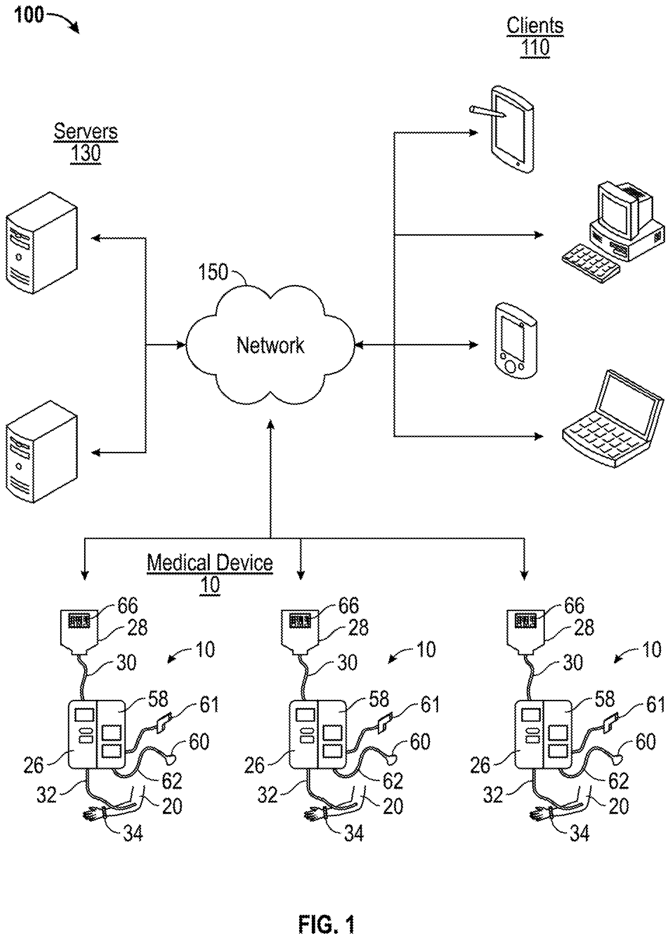

FIG. 1 illustrates an example architecture 100 for monitoring delivery of a fluid medication. The architecture 100 includes medical devices 10, servers 130, and clients 110 connected over a network 150.

Each medical device 10 includes an IV infusion pump 26 and a data reader module 58 for infusing a medication supplied from a hanging medication container 28 through an upper fluid administration line 30 and a lower fluid administration line 32 into a patient 20. In certain embodiments, the medical device 10 is similar to that described in U.S. Pat. No. 5,713,856 to Eggers et Alternatively, other patient care devices, such as pumps, physiological monitors (e.g., heart rate, blood pressure, ECG, EEG, pulse oximeter, and other patient monitors), therapy devices, and other drug delivery devices may be utilized according to the teachings set forth herein.

The data reader module 58 is configured to read bar codes or other information on medication containers with a reader 60 tethered to the module 58 with a wired connection 62. In certain embodiments, a wireless connection can be used instead of wired connection 62. The data reader module 58 is also configured, in certain embodiments, to read radio-frequency identification (RFID) tags on medication containers with a built-in RFID interrogator or RFID reader 61, such as RFID tag label 66 on medication container 28 or a wristband identification tag 34 worn by the patient 20. A data input device other than the bar code reader 60 or RFID reader 61 could be used, such as any device for entering coded data into a computer, such as devices for reading magnetic strips, PCMCIA smart cards, radio frequency cards, memory sticks, CDs, DVDs, or any other analog or digital storage media. Other examples of data input devices include a voice activation or recognition device or a portable personal data assistant (PDA). Additional devices that can be used to identify medication can be found in U.S. Patent Application Publication No. 2006/0229551.

The identifier associated with the patient's identification tag 34 and the identifier associated with the RFID tag label 66 allow the medical device 10 to retrieve information associated with the patient 20 (hereinafter "patient information") and the medication container 28 (hereinafter "medication container information") by referencing the identifiers in a database or other information source that stores the patient information and the medication container information. For example, in certain embodiments, label 66 includes information concerning the contents of the medication container 28. An initial volume of medication in the medication container 28 may also be included in that information. The medical device 10 obtains that volume information either by manual input or by scanning the bar code 66 of the container 28 with the bar code reader 60, or by other means. By any of a number of various methods, the medical device 10 is able to determine the amount of medication remaining to be delivered by the infusion pump 26 during the patient's infusion. The medical device 10 also receives the desired rate of delivery of the medication from reading the label 66 or from manual input as described above and is therefore able to determine the time remaining before the amount of the medication is completely delivered. These determinations by the medical device 10 are readily available, and may be communicated to other devices, such as to the fluid monitoring system, as described below. The patient information and medication container information are jointly referred to as "operating parameters" for the medical device 10 as used herein.

The patient information that is determined by the medical device 10 can include, for example, a patient name, room number or location, identification number, date of birth, bed identifier, height, weight, lists of medications taken or to be taken, associated healthcare providers, allergies, current lab values, vital sign variables being monitored, patient identification number, patient record number, federal identification number, and social security number. The medication container information determined by the medical device 10 can include, for example, a name of a drug in the medication container 28, its concentration, its contraindications, order identification, an associated lab test used to routinely measure therapeutic progress or toxic levels of the medication being described, its cumulative dosing, its twenty-four hour dose rate history, its dose rate, its historical dose rate, its volume rate, its volume remaining, and its status. In certain embodiments, the patient information and/or the medication container information are independently and manually provided, removed, or otherwise edited by a user at the medical device 10. In certain embodiments, patient information and medication container information are retrieved from a server, such as a centralized hospital database that contains patient and medication information, e.g., a medical library database, an alert-discharge-transfer (ADT) system, etc.

The infusion pump 26 of the medical device 10 is configured to provide a value indicating the volume of medication fluid in the medication container 28 and the rate of delivery of the medication fluid in the medication container 28 to the patient 20, which is also included in the medication container information. The infusion pump 26 is capable of being programmed for controlling and monitoring the infusion of the medication fluid to the patient 20.

One or many of the servers 130 can be a hospital information system that is configured to host and track medication order information. The medication order information can include, for example, medication information such as a name of a drug in an ordered medication container, its concentration, its contraindications, order identification, its cumulative dosing guidelines, its twenty-four hour dose rate guideline, its dose rate guidelines, and its volume rate guidelines. The medication order information can also include information on a patient for whom a medication is ordered, such as the patient's name, room number or location, identification number, date of birth, bed identifier, height, weight, lists of medications taken or to be taken, associated healthcare providers, allergies, current lab values, vital sign variables being monitored, patient identification number, patient record number, federal identification number, and social security number. For purposes of load balancing, multiple servers 130 can host the medication order information, either in whole (e.g., as replicated data) or in part. The servers 130 can be any device having an appropriate processor, memory, and communications capability for hosting medication order information.

One or many of the servers 130 can also be a monitoring system for monitoring a delivery of medication in a medication container using a medical device 10 to a patient. The server 130 can include, for example, a monitoring application configured to receive patient information and medication container information from the medical device 10, medication order information from another server 130, and determine or otherwise monitor whether the order for a medication is consistent with an actual medication being delivered to a patient using the medical device 10. For example, the monitoring system may determine that an order for a medication exists for a patient, but the medication is not currently being delivered to the patient. As another example, the monitoring system may determine that an order for a medication does not exist for a patient, but medication is currently being delivered to the patient. If the order for a medication is not consistent with an actual medication being delivered to a patient using the medical device 10, a notification can be sent to a client 110 indicating the lack of consistency. The notification can be an audible notification, a visual notification (e.g., on a mobile device or a nurse viewing station), or other notification configured for notifying a clinician or other responsible caregiver of the lack of consistency between an order for a medication and an actual medication being delivered to a patient using the medical device 10.

The clients 110 to which the servers 130 are connected over the network 150 can be, for example, desktop computers, mobile computers, tablet computers (e.g., including e-book readers), mobile devices (e.g., a smartphone or PDA), or any other devices having appropriate processor, memory, and communications capabilities. The network 150 can include, for example, any one or more of a personal area network (PAN), a local area network (LAN), a campus area network (CAN), a metropolitan area network (MAN), a wide area network (WAN), a broadband network (BBN), the Internet, and the like. Further, the network 150 can include, but is not limited to, any one or more of the following network topologies, including a bus network, a star network, a ring network, a mesh network, a star-bus network, tree or hierarchical network, and the like.

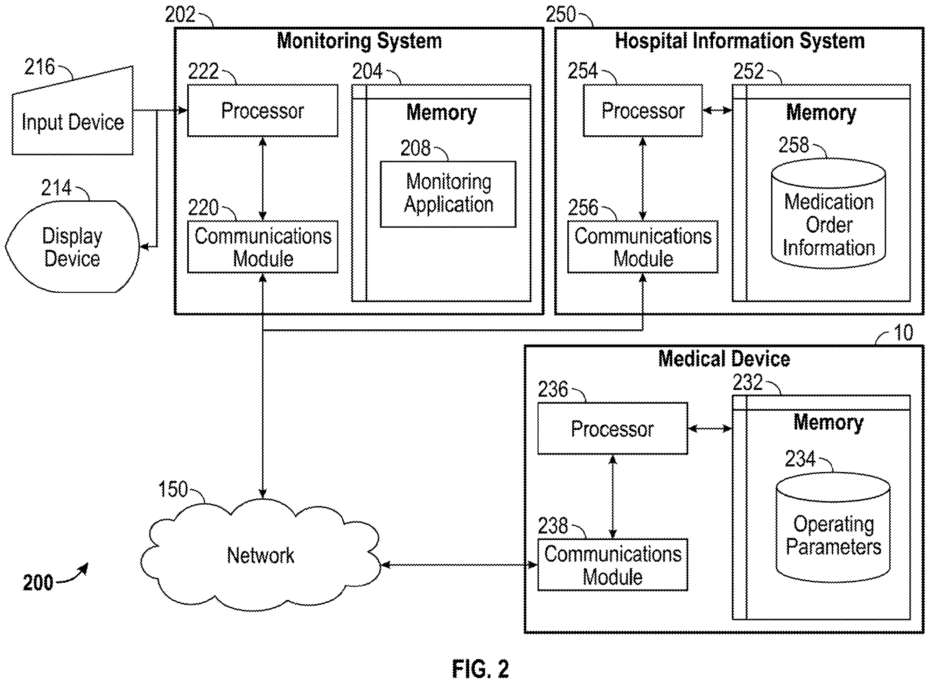

FIG. 2 is a block diagram 200 illustrating an example medical device 10, monitoring system 202, and hospital information system 250 from the architecture 100 of FIG. 1 according to certain aspects of the disclosure. The medical device 10 (e.g., infusion pump), monitoring system 202, and hospital information system 250 are connected over the network 150 via respective communications modules 238, 220, and 256. The communications modules 238, 220, and 256 are configured to interface with the network 150 to send and receive information, such as data, requests, responses, and commands to other devices on the network. The communications modules 238, 220, and 256 can be, for example, modems or Ethernet cards.

The monitoring system 202 includes a processor 222, a communications module 220, and a memory 204 that includes a monitoring application 208. The monitoring application can be configured by a user, for example, using an input device 216 (e.g., keyboard) and a display device 214 (e.g., electronic display). The processor 222 of the monitoring system 202 is configured to execute instructions, such as instructions physically coded into the processor 236, instructions received from software (e.g., monitoring application 208) in memory 240, or a combination of both. For example, the processor 236 of the monitoring system 202 executes instructions to receive first information indicating an order for a fluid medication in a container for a patient, and second information indicating a current status of delivery of fluid medication to the patient.

The first information indicating the order for a fluid medication in a container can be received from the hospital information system 250 (e.g., which tracks medication orders for patients) over the network 150 using respective communications modules 256 and 220 of the hospital information system 250 and monitoring system 202, respectively. Specifically, in response to a request for the first information sent by the processor 222 of the monitoring system 202, the processor 254 of the hospital information system 250 provides to the monitoring system 202 medication order information 258 from a memory 252 of the hospital information system 250. The medication order information 258 includes, for example, medication information and information on a patient for whom the medication is prescribed.

The second information indicating the current status of delivery of fluid medication to the patient can be received from the medical device 10 (e.g., a fluid delivery system) over the network 150 using respective communications modules 238 and 220 of the medical device 10 and monitoring system 202, respectively. Specifically, in response to a request for the second information sent by the processor 222 of the monitoring system 202, the processor 236 of the medical device 10 provides to the monitoring system 202 operating parameters 234 for the medical device 10 from a memory 232 of the medical device 10. The operating parameters 234 for the medical device 10 include, for example, medication container information and patient information for the medication container being provided to the patient using the medical device 10. As discussed above, the patient information can include, for example, a patient name, room number or location, identification number, date of birth, bed identifier, height, weight, lists of medications taken or to be taken, associated healthcare providers, allergies, current lab values, vital sign variables being monitored, patient identification number, patient record number, federal identification number, and social security number. The medication container information determined by the medical device 10 can include, for example, a name of a drug in the medication container 28, its concentration, its contraindications, order identification, an associated lab test used to routinely measure therapeutic progress or toxic levels of the medication being described, its cumulative dosing, its twenty-four hour dose rate history, its current dose rate (e.g., by medical device 10), its historical dose rate, its volume rate (e.g., by medical device 10), its volume remaining (e.g., by medical device 10), and its status (e.g., by medical device 10).

In certain aspects where the patient associated with a current status of delivery of fluid medication is not identified, such as where a nurse or other caregiver forgets to identify the patient to the medical device 10 when initiating the delivery of the fluid medication, a notification may be displayed on the display device 214 of the monitoring system 202. The notification, when selected, may display a location of the medical device 10 within an institution based on the serial number of the medical device 10 and information indicating where the medical device 10 having that serial number is located. A notification may then be sent to an administrator, other caregiver, or device at or near the location of the medical device 10 indicating that the patient associated with the medical device 10 should be identified. For example, the display device 214 can display the notification message "unknown" where a patient's name would typically be displayed. Upon clicking or touching "unknown," a map of the institution may be provided that identifies where the medical device 10 with the unknown patient is located. An option may then be presented to send an alert to the location of the medical device 10 indicating a need to identify the patient.

The processor 236 of the monitoring system 202 also executes instructions to determine whether the order for the fluid medication for the patient (e.g., as indicated by the first information from the hospital information system 250) is consistent with the current status of delivery of fluid medication (e.g., as indicated by the second information from the medical device 10) to the patient. The processor 236 is configured to provide an alert indicating an inconsistency between the order for the fluid medication and the current status of delivery of fluid medication when the order for the fluid medication for the patient is inconsistent with the delivery of fluid medication to the patient. The alert can be, for example, a visual and/or audible alert displayed on the hospital information system 250, medical device 10, a device (e.g., smartphone) of a caregiver responsible for the patient, or other device. The alert can also be, for example, a message such as an email or text message that is sent to a device of the caregiver responsible for the patient, or a request or command to another system to conduct analysis for further clinical decision making..

The order for the fluid medication for the patient can be inconsistent with the delivery of fluid medication where, for example, no current order for fluid medication for the patient exists yet fluid medication is currently being delivered to the patient. For instance, a patient may currently be receiving adrenaline at the medical device 10, but no order for adrenaline exists for the patient in the medication order information 258.

The order for the fluid medication for the patient can also be inconsistent with the delivery of fluid medication when, for example, no fluid medication is being delivered to the patient yet there is a current order for fluid medication for the patient. For instance, a patient may, according to medication order information 258, be prescribed to currently receive adrenaline at the medical device 10, but the patient is not receiving adrenaline at medical device 10.

In certain aspects, the order for the fluid medication for the patient can further be inconsistent with the delivery of fluid medication when, for example, there is an inconsistency in delivery parameters between the order for the fluid medication for the patient (e.g., according to medication order information 258) and the delivery of fluid medication to the patient (e.g., according to the operating parameters 234 of the medical device 10). For instance, a patient according to medication order information 258 may be prescribed to currently receive adrenaline at a rate of 1 mcg/min, but may currently be receiving adrenaline at the medical device 10 at a rate of 4 mcg/min.

In certain aspects, the first information is received from an external data system (e.g., hospital information system 250) in a native message format of the external data system, and the processor 222 of the monitoring system 202 can be configured to convert the first information into an internal messaging format configured for use with the monitoring system 202. Similarly, the second information is received from another external data system (e.g., medical device 10) in a native message format of the external data system, and the processor 222 of the monitoring system 202 can be configured to convert the second information into the internal messaging format configured for use with the monitoring system 202. The processor 212 can be configured to perform the conversion according to the system and method of converting messages being sent between data systems using different communication protocols and message structures described in U.S. Pat. App. No. 13/421,776, entitled "Scalable Communication System," and filed on March 15, 2012, The memory 204 of the monitoring system 202 can include, for example, an interface module for communicating with the server 130. The interface module can include information on the communication protocol and data structure used by the server 130 and is configured to both receive messages from and transmit messages to the server 130.

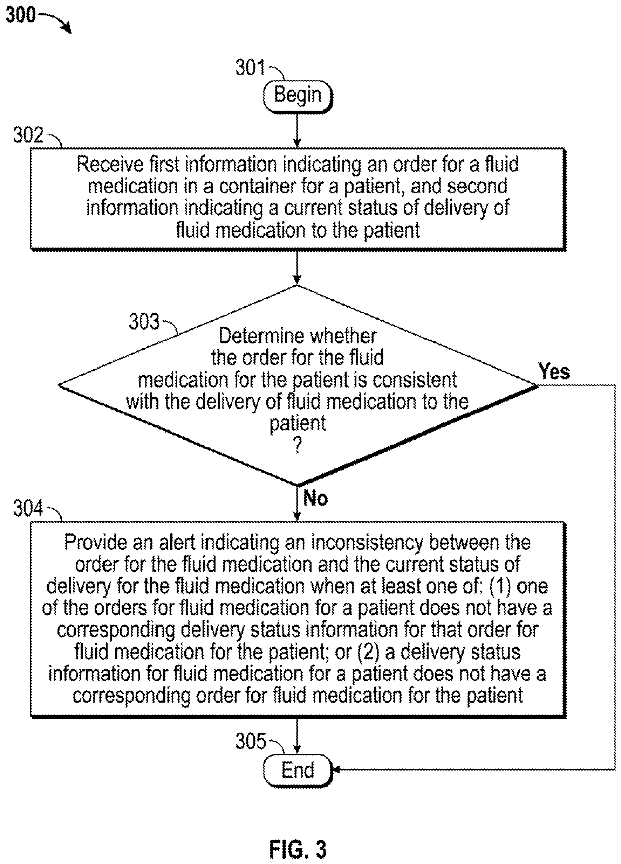

FIG. 3 illustrates an example process 300 for monitoring delivery of a medication using the example monitoring system 202 of FIG. 2. While FIG. 3 is described with reference to FIG. 2, it should be noted that the process steps of FIG. 3 may be performed by other systems. The process 300 begins by proceeding from beginning step 301 when a monitoring system 202 is activated or otherwise instructed to monitor delivery of a medication, to step 302 when first information (e.g., from medication order information 258 on the hospital information system 250) indicating an order for a fluid medication in a container for a patient, and second information (e.g., from operating parameters 234 on the medical device 10) indicating a current status of delivery of fluid medication to the patient, are received (e.g., by monitoring system 202). In decision step 303, a determination is made whether the order for the fluid medication for the patient is consistent with the current status of delivery of fluid medication to the patient.

If the determination of decision step 303 indicates that the order for the fluid medication for the patient is consistent with the current status of delivery of fluid medication to the patient, the process 300 proceeds to and ends at step 305. If, however, the determination of decision step 303 indicates that the order for the fluid medication for the patient is not consistent with the current status of delivery of fluid medication to the patient, the process 300 proceeds to step 304. In step 304, an alert is provided (e.g., to medical device 10, hospital information system 250, a caregiver's device, or other device) indicating an inconsistency between the order for the fluid medication and the current status of delivery of fluid medication when one of the orders for fluid medication for a patient does not have a corresponding delivery status information for that order for fluid medication for the patient, or a delivery status information for fluid medication for a patient does not have a corresponding order for fluid medication for that patient. The process 300 then ends in step 305.

FIG. 3 set forth an example process 300 for monitoring delivery of a medication using the example monitoring system 202 of FIG. 2. An example will now be described using the example process 300 of FIG. 3, a medical device 10 that is an infusion pump, and a nurse viewer device configured to receive notifications.

The process 300 begins by proceeding from beginning step 301 when a monitoring system 202 is activated or otherwise instructed to monitor delivery of a medication, to step 302 when the monitoring system 202 receives medication order information 258 from the hospital information system 250 indicating three orders for fluid medications in containers for three separate patients, and operating parameters 234 from each infusion pump 10 for each of the three separate patients indicating a current status of delivery of fluid medication to the patient. In decision step 303, the monitoring system 202 analyzes the three orders for fluid medications and the current status of delivery of fluid medication to each patient, and determines that medication orders for two of the three fluid medication containers and are not consistent with the current delivery of fluid medication to the patients at their respective infusion pumps 10, and the process proceeds to step 304.

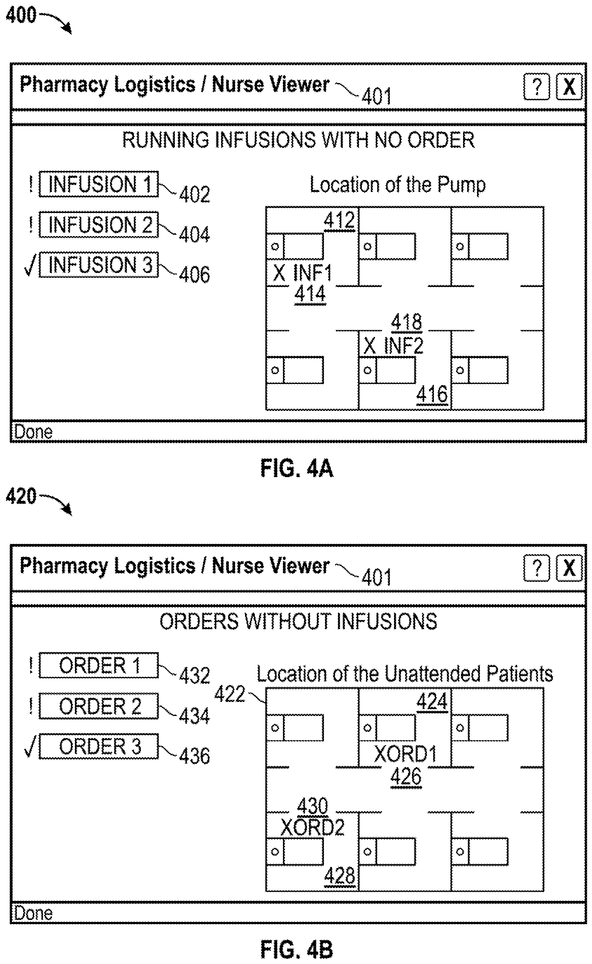

In step 304, an alert is provided to a nurse viewer 401 as provided in the example illustration 400 of FIG. 4A. The illustration of FIG. 4A particularly displays a graphical user interface of a nurse viewer that alerts currently running infusions that do not have existing orders for the infusions. Specifically, the graphical user interface identifies an alert for "infusion I" 402 and "infusion 2" 404 as denoted by the "!" symbol, thereby indicating that there are no medication orders for "infusion 1" 402 and "infusion 2" 404, yet infusions are being provided to the patients associated with "infusion 1" 402 and "infusion 2" 404. The graphical user interface also identifies, with a check symbol, that there is a medication order for "infusion 3" 406, and that medication order is properly being delivered to the patient associated with "infusion 3" 406. The graphical user interface also includes a map 410 identifying the location of each infusion pump 10 within the corresponding healthcare facility. Specifically, the map 410 indicates an alert 414 for an illustrated patient room 412 associated with "infusion I," and also indicates an alert 418 for an illustrated patient room 416 associated with "infusion 2."

In certain aspects, as provided in the example illustration 420 of FIG. 4B, the graphical user interface of the nurse viewer 401 can provide alerts for existing orders of infusions for patients that currently are not receiving any infusions. Specifically, the graphical user interface identifies an alert for "order 1" 432 and "order 2" 434 as denoted by the "!" symbol, thereby indicating that there are existing orders for infusions for the two patients associated with "order 1" 432 and "order 2" 434, yet no infusions are being provided to those two patients. The graphical user interface also identifies, with a check symbol, that there is a medication order for "order 3" 436, and that medication order is properly being delivered to the patient associated with "order 3" 436. The graphical user interface also includes a map 422 identifying the location of each infusion pump 10 within the corresponding healthcare facility. Specifically, the map 422 indicates an alert 426 for an illustrated patient room 424 associated with "order 1," and also indicates an alert 430 for an illustrated patient room 428 associated with "order 2."

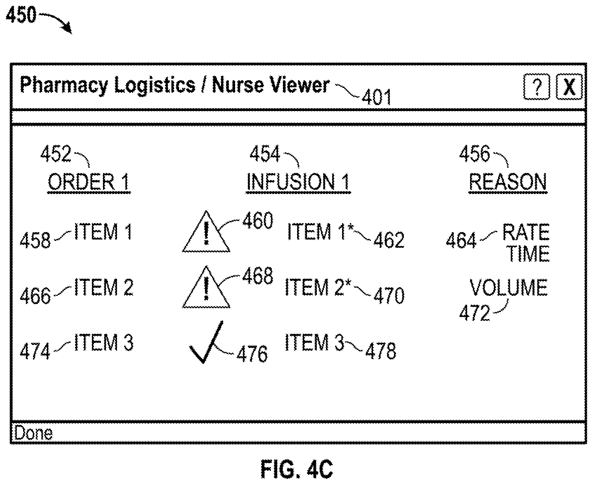

In certain other aspects, as provided in the example illustration 450 of FIG. 4C, the graphical user interface of the nurse viewer 401 can provide alerts for inconsistencies between existing orders of infusions for patients and infusions currently being provided to those patients. The graphical user interface identifies medication orders 452, infusions 454 associated with the orders, and any reason 456 for an inconsistency between the medication order 452 and the associated current infusion 454. The graphical user interface, for example, identifies an alert 460 for "item 1" 458 denoted by the "!" symbol, thereby indicating that the existing order for an infusion for the patient associated with "item 1" 458 is not consistent with the infusion currently being provided to the patient as "item 1" 462, and further indicates that the reason 464 for the inconsistency is the rate time of the infusion of "item 1" 462. The graphical user interface also identifies an alert 468 for "item 2" 466 denoted by the "!" symbol, thereby indicating that the existing order for an infusion for the patient associated with "item 2" 466 is not consistent with the infusion currently being provided to the patient as "item 2" 470, and further indicates that the reason 472 for the inconsistency is the volume of the infusion of "item 2" 470. The graphical user interface further identifies, using a check symbol 476 for "item 3" 474, that the existing order for an infusion for the patient associated with "item 3" 476 is consistent with the infusion currently being provided to the patient as "item 3" 478.

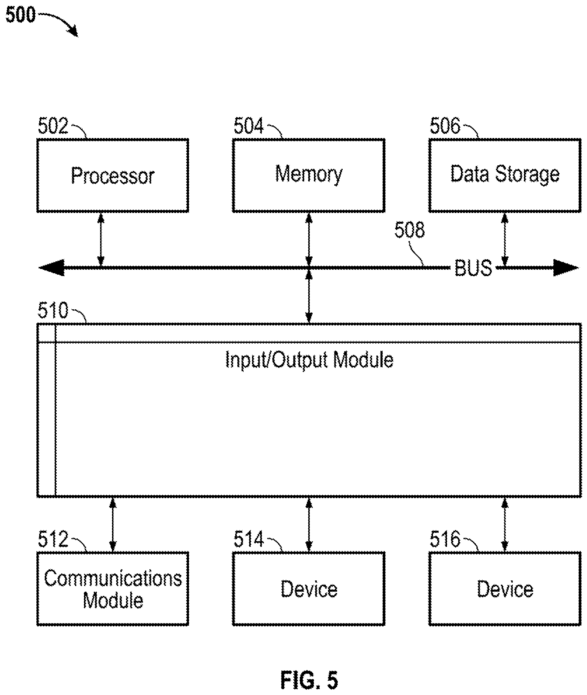

FIG. 5 is a block diagram illustrating an example computer system 500 with which the monitoring system 202, the hospital information system 250, and the medical device 10 of FIG. 2 can be implemented. In certain aspects, the computer system 500 may be implemented using hardware or a combination of software and hardware, either in a dedicated server, or integrated into another entity, or distributed across multiple entities.

Computer system 500 (e.g., the monitoring system 202, the hospital information system 250, and the medical device 10) includes a bus 508 or other communication mechanism for communicating information, and a processor 502 (e.g., processor 222, 254, and 236) coupled with bus 508 for processing information. By way of example, the computer system 500 may be implemented with one or more processors 502. Processor 502 may be a general-purpose microprocessor, a microcontroller, a Digital Signal Processor (DSP), an Application Specific Integrated Circuit (ASIC), a Field Programmable Gate Array (FPGA), a Programmable Logic Device (PLD), a controller, a state machine, gated logic, discrete hardware components, or any other suitable entity that can perform calculations or other manipulations of information.

Computer system 500 can include, in addition to hardware, code that creates an execution environment for the computer program in question, e.g., code that constitutes processor firmware, a protocol stack, a database management system, an operating system, or a combination of one or more of them stored in an included memory 504 (e.g., memory 204, 252, and 232), such as a Random Access Memory (RAM), a flash memory, a Read Only Memory (ROM), a Programmable Read-Only Memory (PROM), an Erasable PROM (EPROM), registers, a hard disk, a removable disk, a CD-ROM, a DVD, or any other suitable storage device, coupled to bus 508 for storing information and instructions to be executed by processor 502. The processor 502 and the memory 504 can be supplemented by, or incorporated in, special purpose logic circuitry.

The instructions may be stored in the memory 504 and implemented in one or more computer program products, i.e., one or more modules of computer program instructions encoded on a computer readable medium for execution by, or to control the operation of, the computer system 500, and according to any method well known to those of skill in the art, including, but not limited to, computer languages such as data-oriented languages (e.g., SQL, dBase), system languages (e.g., C, Objective-C, C++, Assembly), architectural languages (e.g., Java, .NET), and application languages (e.g., PHP, Ruby, Perl, Python). Instructions may also be implemented in computer languages such as array languages, aspect-oriented languages, assembly languages, authoring languages, command line interface languages, compiled languages, concurrent languages, curly-bracket languages, dataflow languages, data-structured languages, declarative languages, esoteric languages, extension languages, fourth-generation languages, functional languages, interactive mode languages, interpreted languages, iterative languages, list-based languages, little languages, logic-based languages, machine languages, macro languages, metaprogramming languages, multiparadigm languages, numerical analysis, non-English-based languages, object-oriented class-based languages, object-oriented prototype-based languages, off-side rule languages, procedural languages, reflective languages, rule-based languages, scripting languages, stack-based languages, synchronous languages, syntax handling languages, visual languages, wirth languages, embeddable languages, and xml-based languages. Memory 504 may also be used for storing temporary variable or other intermediate information during execution of instructions to be executed by processor 502.

A computer program as discussed herein does not necessarily correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, subprograms, or portions of code). A computer program can be deployed to be executed on one computer or on multiple computers that are located at one site or distributed across multiple sites and interconnected by a communication network. The processes and logic flows described in this specification can be performed by one or more programmable processors executing one or more computer programs to perform functions by operating on input data and generating output.

Computer system 500 further includes a data storage device 506 such as a magnetic disk or optical disk, coupled to bus 508 for storing information and instructions. Computer system 500 may be coupled via input/output module 510 to various devices. The input/output module 510 can be any input/output module. Example input/output modules 510 include data ports such as USB ports. The input/output module 510 is configured to connect to a communications module 512. Example communications modules 512 (e.g., communications module 220, 256, and 238) include networking interface cards, such as Ethernet cards and modems. In certain aspects, the input/output module 510 is configured to connect to a plurality of devices, such as an input device 514 (e.g., input device 216) and/or an output device 516 (e.g., display device 214). Example input devices 514 include a keyboard and a pointing device, e.g., a mouse or a trackball, by which a user can provide input to the computer system 500. Other kinds of input devices 514 can be used to provide for interaction with a user as well, such as a tactile input device, visual input device, audio input device, or brain-computer interface device. For example, feedback provided to the user can be any form of sensory feedback, e.g., visual feedback, auditory feedback, or tactile feedback; and input from the user can be received in any form, including acoustic, speech, tactile, or brain wave input. Example output devices 516 include display devices, such as a LED (light emitting diode), CRT (cathode ray tube), or LCD (liquid crystal display) screen, for displaying information to the user.

According to one aspect of the present disclosure, the monitoring system 202, the hospital information system 250, and the medical device 10 can be implemented using a computer system 500 in response to processor 502 executing one or more sequences of one or more instructions contained in memory 504. Such instructions may be read into memory 504 from another machine-readable medium, such as data storage device 506. Execution of the sequences of instructions contained in main memory 504 causes processor 502 to perform the process steps described herein. One or more processors in a multi-processing arrangement may also be employed to execute the sequences of instructions contained in memory 504. In alternative aspects, hard-wired circuitry may be used in place of or in combination with software instructions to implement various aspects of the present disclosure. Thus, aspects of the present disclosure are not limited to any specific combination of hardware circuitry and software.

Various aspects of the subject matter described in this specification can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the subject matter described in this specification, or any combination of one or more such back end, middleware, or front end components. The components of the system can be interconnected by any form or medium of digital data communication, e.g., a communication network. The communication network (e.g., network 150) can include, for example, any one or more of a personal area network (PAN), a local area network (LAN), a campus area network (CAN), a metropolitan area network (MAN), a wide area network (WAN), a broadband network (BBN), the Internet, and the like. Further, the communication network can include, but is not limited to, for example, any one or more of the following network topologies, including a bus network, a star network, a ring network, a mesh network, a star-bus network, tree or hierarchical network, or the like. The communications modules can be, for example, modems or Ethernet cards.

Computing system 500 can include clients and servers. A client and server are generally remote from each other and typically interact through a communication network. The relationship of client and server arises by virtue of computer programs running on the respective computers and having a client-server relationship to each other. Computer system 500 can be, for example, and without limitation, a desktop computer, laptop computer, or tablet computer. Computer system 500 can also be embedded in another device, for example, and without limitation, a mobile telephone, a personal digital assistant (PDA), a mobile audio player, a Global Positioning System (GPS) receiver, a video game console, and/or a television set top box.

The term "machine-readable storage medium" or "computer readable medium" as used herein refers to any medium or media that participates in providing instructions or data to processor 502 for execution. Such a medium may take many forms, including, but not limited to, non-volatile media, volatile media, and transmission media. Non-volatile media include, for example, optical disks, magnetic disks, or flash memory, such as data storage device 506. Volatile media include dynamic memory, such as memory 504. Transmission media include coaxial cables, copper wire, and fiber optics, including the wires that comprise bus 508. Common forms of machine-readable media include, for example, floppy disk, a flexible disk, hard disk, magnetic tape, any other magnetic medium, a CD-ROM, DVD, any other optical medium, punch cards, paper tape, any other physical medium with patterns of holes, a RAM, a PROM, an EPROM, a FLASH EPROM, any other memory chip or cartridge, or any other medium from which a computer can read. The machine-readable storage medium can be a machine-readable storage device, a machine-readable storage substrate, a memory device, a composition of matter effecting a machine-readable propagated signal, or a combination of one or more of them.

As used herein, the phrase "at least one of" preceding a series of items, with the terms "and" or "or" to separate any of the items, modifies the list as a whole, rather than each member of the list (i.e., each item). The phrase "at least one of" does not require selection of at least one item; rather, the phrase allows a meaning that includes at least one of any one of the items, and/or at least one of any combination of the items, and/or at least one of each of the items. By way of example, the phrases "at least one of A, B, and C" or "at least one of A, B, or C" each refer to only A, only B, or only C; any combination of A, B, and C; and/or at least one of each of A, B, and C.

Furthermore, to the extent that the term "include," "have," or the like is used in the description or the claims, such term is intended to be inclusive in a manner similar to the term "comprise" as "comprise" is interpreted when employed as a transitional word in a claim.

A reference to an element in the singular is not intended to mean "one and only one" unless specifically stated, but rather "one or more." All structural and functional equivalents to the elements of the various configurations described throughout this disclosure that are known or later come to be known to those of ordinary skill in the art are expressly incorporated herein by reference and intended to be encompassed by the subject technology. Moreover, nothing disclosed herein is intended to be dedicated to the public regardless of whether such disclosure is explicitly recited in the above description.

While this specification contains many specifics, these should not be construed as limitations on the scope of what may be claimed, but rather as descriptions of particular implementations of the subject matter. Certain features that are described in this specification in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the aspects described above should not be understood as requiring such separation in all aspects, and it should be understood that the described program components and systems can generally be integrated together in a single software product or packaged into multiple software products.

The subject matter of this specification has been described in terms of particular aspects, but other aspects can be implemented and are within the scope of the following claims. For example, the actions recited in the claims can be performed in a different order and still achieve desirable results. As one example, the processes depicted in the accompanying figures do not necessarily require the particular order shown, or sequential order, to achieve desirable results. In certain implementations, multitasking and parallel processing may be advantageous. Other variations are within the scope of the following claims.

These and other implementations are within the scope of the following claims.

## Claims

1. A fluid medication delivery monitoring system (100,202) comprising:
a memory (204) configured to receive first information indicating orders for infusion of fluid medications for patients (258), and second information indicating delivery status information for infusion of fluid medications for patients (234);
a processor (222) configured to:
compare the orders for infusion of fluid medications for patients with the delivery status information for infusion of fluid medications for patients; and
provide a notification when the comparison shows at least one of:
an inconsistency between one of the orders for the infusion of the fluid medication according to the first information and the delivery status information for the infusion of the fluid medication according to operating parameters of a medical device for the infusion; or
a delivery status information for infusion of fluid medication for a patient does not have a corresponding order for infusion of fluid medication for that patient,
wherein the second information is received from the medical device, and
wherein the medical device comprises an infusion pump.

2. The system of Claim 1, wherein the first information comprises at least one of a medication name, medication amount, rate of medication delivery, patient name, and caregiver name.

3. The system of Claim 2, wherein the first information is received from a hospital information system.

4. The system of Claim 1, wherein the second information comprises at least one of a current amount of medication being provided to a patient, a rate of medication delivery of the medication being provided to the patient, a name of the medication being provided to the patient, or a name of a caregiver providing the medication to the patient.

5. The system of Claim 1, wherein the first information is received over a network from a healthcare facility information system in a first native message format of the healthcare facility information system, and the second information is received over the network from the medical device in a second native message format of the medical device, and wherein the first information and the second information is converted into an internal messaging format configured for use with the monitoring system.

6. The system of Claim 1, wherein the first information is received from a medication order tracking system, and the infusion pump comprises a fluid delivery system.

7. A computer-implemented method for monitoring delivery of a fluid medication, the method comprising:
receiving first information indicating orders for infusion of fluid medications for patients, and second information indicating delivery status information for infusion of fluid medications for patients;
comparing the orders for infusion of fluid medications for patients with the delivery status information for infusion of fluid medications for patients; and
providing a notification when the comparison shows at least one of:
an inconsistency between one of the orders for the infusion of the fluid medication according to the first information and the delivery status information for the infusion of the fluid medication according to operating parameters of a medical device for the infusion; or
a delivery status information for infusion of fluid medication for a patient does not have a corresponding order for infusion of fluid medication for that patient,
wherein the second information is received from a medical device, and
wherein the medical device comprises an infusion pump.

8. The method of Claim 7, wherein the first information comprises at least one of a medication name, medication amount, rate of medication delivery, patient name, and caregiver name.

9. The method of Claim 8, wherein the first information is received from a hospital information system.

10. The method of Claim 7, wherein the second information comprises at least one of a current amount of medication being provided to a patient, a rate of medication delivery of the medication of the medication being provided to the patient, a name of the medication being provided to the patient, or a name of a caregiver providing the medication to the patient.

11. The method of Claim 7,
wherein the notification is provided by a monitoring system,
wherein the first information is received over a network from a healthcare facility information system in a first native message format of the healthcare facility information system, and the second information is received over the network from the medical device in a second native message format of the medical device, and
wherein the first information and the second information is converted into an internal messaging format configured for use with the monitoring system.

12. The method of Claim 7, wherein the first information is received from a medication order tracking system, and the infusion pump comprises a fluid delivery system.

13. A machine-readable storage medium comprising machine-readable instructions for causing a processor, when executed, to execute a method for monitoring delivery of a fluid medication, the method comprising:
receiving first information indicating orders for infusion of fluid medications for patients, and second information indicating delivery status information for infusion of fluid medications for patients;
comparing the orders for infusion of fluid medications for patients with the delivery status information for infusion of fluid medications for patients; and
providing a notification when the comparison shows at least one of:
an inconsistency between one of the orders for the infusion of the fluid medication according to the first information and the delivery status information for the infusion of the fluid medication according to operating parameters of a medical device for the infusion; or
a delivery status information for infusion of fluid medication for a patient does not have a corresponding order for infusion of fluid medication for that patient,
wherein the second information is received from a medical device, and
wherein the medical device comprises an infusion pump.

14. The machine-readable storage medium of Claim 13, wherein the first information comprises at least one of a medication name, medication amount, rate of medication delivery, patient name, and caregiver name.

15. The machine-readable storage medium of Claim 13, wherein the second information comprises at least one of a current amount of medication being provided to a patient, a rate of medication delivery of the medication being provided to the patient, a name of the medication being provided to the patient, or a name of a caregiver providing the medication to the patient.

16. The method of Claim 7, the method further comprising:
receiving an indication that a patient associated with the medical device is unidentified;
providing a notification indicating a location of the medical device in the institution;
sending, to a display associated with the medical device, a request to identify the patient associated with the medical device; and
receiving an identification of the patient associated with the medical device.

## Patentansprüche

1. Überwachungssystem für die Abgabe eines flüssigen Medikaments (100, 202), umfassend:
einen Speicher (204),
konfiguriert zum Empfangen einer ersten Information, die die Aufträge für die Infusion von flüssigen Medikamenten für Patienten (258) anzeigt,
und einer zweiten Information, die eine Abgabestatusinformation über die Infusion von flüssigen Medikamenten für Patienten (234) anzeigt;
einen Prozessor (222), der zu Folgendem konfiguriert ist:
Vergleichen der Aufträge für die Infusion von flüssigen Medikamenten für Patienten mit der Abgabestatusinformation über die Infusion von flüssigen Medikamenten für Patienten; und
Bereitstellen einer Benachrichtigung, wenn der Vergleich mindestens eines der folgenden zeigt:
eine fehlende Übereinstimmung zwischen einem der Aufträge für die Infusion des flüssigen Medikaments gemäß der ersten Information und der Abgabestatusinformation über die Infusion des flüssigen Medikaments gemäß der Betriebsparameter einer medizinischen Vorrichtung für die Infusion; oder
für eine Abgabestatusinformation über die Infusion des flüssigen Medikaments für einen Patienten besteht kein übereinstimmender Auftrag bezüglich der Infusion des flüssigen Medikaments für diesen Patienten,
wobei die zweite Information von der medizinischen Vorrichtung empfangen wird, und
wobei die medizinische Vorrichtung eine Infusionspumpe umfasst.

2. System nach Anspruch 1, wobei die erste Information mindestens eines von einer Bezeichnung des Medikaments, einer Medikamentenmenge, einer Abgaberate des Medikaments, einem Namen des Patienten und einem Namen eines Pflegers umfasst.

3. System nach Anspruch 2, wobei die erste Information von einem Krankenhausinformationssystem empfangen wird.

4. System nach Anspruch 1, wobei die zweite Information mindestes eines von einer derzeitigen Menge des an einen Patienten verabreichten Medikaments, einer Medikamentenabgaberate des an den Patienten abgegebenen Medikaments, einer Bezeichnung des an den Patienten verabreichten Medikaments oder eines Namens eines Pflegers umfasst, der das Medikament an den Patienten verabreicht.

5. System nach Anspruch 1, wobei die erste Information über ein Netzwerk von einem Informationssystem einer Gesundheitseinrichtung in einem ersten nativen Nachrichtenformat des Informationssystems einer Gesundheitseinrichtung empfangen wird, und die zweite Information über das Netzwerk von der medizinischen Vorrichtung in einem zweiten nativen Nachrichtenformat der medizinischen Vorrichtung empfangen wird, und wobei die erste Information und die zweite Information in ein internes Nachrichtenübermittlungsformat konvertiert werden, das zur Verwendung mit dem Überwachungssystem konfiguriert ist.

6. System nach Anspruch 1, wobei die erste Information von einem Medikamentenauftrag-Trackingsystem empfangen wird und die Infusionspumpe ein Flüssigkeitsabgabesystem umfasst.

7. Computerimplementiertes Verfahren zum Überwachen der Abgabe eines flüssigen Medikaments, wobei das Verfahren Folgendes umfasst:
Empfangen einer ersten Information, die die Aufträge für die Infusion von flüssigen Medikamenten für Patienten anzeigt, und einer zweiten Information, die eine Abgabestatusinformation über die Infusion von flüssigen Medikamenten für Patienten anzeigt;
Vergleichen der Aufträge für die Infusion von flüssigen Medikamenten für Patienten mit der Abgabestatusinformation über die Infusion von flüssigen Medikamenten für Patienten; und
Bereitstellen einer Benachrichtigung, wenn der Vergleich mindestens eines der folgenden zeigt:
eine fehlende Übereinstimmung zwischen einem der Aufträge für die Infusion des flüssigen Medikaments gemäß der ersten Information und der Abgabestatusinformation über die Infusion des flüssigen Medikaments gemäß der Betriebsparameter einer medizinischen Vorrichtung für die Infusion; oder
für eine Abgabestatusinformation über die Infusion des flüssigen Medikaments für einen Patienten besteht kein übereinstimmender Auftrag bezüglich der Infusion des flüssigen Medikaments für diesen Patienten,
wobei die zweite Information von der medizinischen Vorrichtung empfangen wird, und
wobei die medizinische Vorrichtung eine Infusionspumpe umfasst.

8. Verfahren nach Anspruch 7, wobei die erste Information mindestens eines von einer Bezeichnung des Medikaments, einer Medikamentenmenge, einer Abgaberate des Medikaments, einem Namen des Patienten und einem Namen eines Pflegers umfasst.

9. Verfahren nach Anspruch 8, wobei die erste Information von einem Krankenhausinformationssystem empfangen wird.

10. Verfahren nach Anspruch 7, wobei die zweite Information mindestes eines von einer derzeitigen Menge des an einen Patienten verabreichten Medikaments, einer Medikamentenabgaberate des an den Patienten abgegebenen Medikaments, einer Bezeichnung des an den Patienten verabreichten Medikaments oder eines Namens eines Pflegers umfasst, der das Medikament an den Patienten verabreicht.

11. Verfahren nach Anspruch 7,
wobei die Benachrichtigung von einem Überwachungssystem bereitgestellt wird,
wobei die erste Information über ein Netzwerk von einem Informationssystem einer Gesundheitseinrichtung in einem ersten nativen Nachrichtenformat des Informationssystems einer Gesundheitseinrichtung empfangen wird, und die zweite Information über das Netzwerk von der medizinischen Vorrichtung in einem zweiten nativen Nachrichtenformat der medizinischen Vorrichtung empfangen wird, und
wobei die erste Information und die zweite Information in ein internes Nachrichtenübermittlungsformat konvertiert werden, das zur Verwendung mit dem Überwachungssystem konfiguriert ist.

12. Verfahren nach Anspruch 7, wobei die erste Information von einem Medikamentenauftrag-Trackingsystem empfangen wird und die Infusionspumpe ein Flüssigkeitsabgabesystem umfasst.

13. Maschinenlesbares Speichermedium, umfassend maschinenlesbare Anweisungen, um einen Prozessor bei Ausführung zu Folgendem zu veranlassen:
Ausführen eines Verfahrens zum Überwachen der Abgabe eines flüssigen Medikaments, wobei das Verfahren Folgendes umfasst:
Empfangen einer ersten Information, die die Aufträge für die Infusion von flüssigen Medikamenten für Patienten anzeigt, und einer zweiten Information, die eine Abgabestatusinformation über die Infusion von flüssigen Medikamenten für Patienten anzeigt;
Vergleichen der Aufträge für die Infusion von flüssigen Medikamenten für Patienten mit der Abgabestatusinformation über die Infusion von flüssigen Medikamenten für Patienten; und
Bereitstellen einer Benachrichtigung, wenn der Vergleich mindestens eines der folgenden zeigt:
eine fehlende Übereinstimmung zwischen einem der Aufträge für die Infusion des flüssigen Medikaments gemäß der ersten Information und der Abgabestatusinformation über die Infusion des flüssigen Medikaments gemäß der Betriebsparameter einer medizinischen Vorrichtung für die Infusion; oder
für eine Abgabestatusinformation über die Infusion des flüssigen Medikaments fürs einen Patienten besteht kein übereinstimmender Auftrag bezüglich der Infusion des flüssigen Medikaments für diesen Patienten,
wobei die zweite Information von der medizinischen Vorrichtung empfangen wird, und
wobei die medizinische Vorrichtung eine Infusionspumpe umfasst.

14. Maschinenlesbares Speichermedium nach Anspruch 13, wobei die erste Information mindestens eines von einer Bezeichnung des Medikaments, einer Medikamentenmenge, einer Abgaberate des Medikaments, einem Namen des Patienten und einem Namen eines Pflegers umfasst.

15. Maschinenlesbares Speichermedium nach Anspruch 13, wobei die zweite Information mindestes eines von einer derzeitigen Menge des an einen Patienten verabreichten Medikaments, einer Medikamentenabgaberate des an den Patienten abgegebenen Medikaments, einer Bezeichnung des an den Patienten verabreichten Medikaments oder eines Namens eines Pflegers umfasst, der das Medikament an den Patienten verabreicht.

16. Verfahren nach Anspruch 7, wobei das Verfahren ferner Folgendes umfasst:
Empfangen einer Anzeige, dass ein Patient, der mit der medizinischen Vorrichtung assoziiert ist, nicht identifiziert ist;
Bereitstellen einer Benachrichtigung, die einen Standort der medizinischen Vorrichtung in der Einrichtung anzeigt;
Senden einer Anfrage zur Identifizierung des Patienten, der mit der medizinischen Vorrichtung assoziiert ist, an einen mit der medizinischen Vorrichtung assoziierten Bildschirm; und Empfangen einer Identifizierung des mit der medizinischen Vorrichtung assoziierten Patienten.

## Revendications

1. Système de contrôle d'administration de médicament liquide (100, 202) comprenant :
- une mémoire (204) destinée à recevoir des premières informations indiquant des prescriptions de perfusion de médicaments liquides pour patients (258),
- et des secondes informations indiquant des informations sur le statut d'administration de perfusion de médicaments liquides pour patients (234) ;
- une unité centrale (222) destinée à :
- comparer les prescriptions de perfusion de médicaments liquides pour patients aux informations sur le statut d'administration de perfusion de médicaments liquides pour patients ; et
- fournir une notification lorsque la comparaison montre au moins :
- une incohérence entre l'une des prescriptions de la perfusion du médicament liquide selon la première information et l'information sur le statut d'administration de la perfusion du médicament liquide selon les paramètres d'exploitation d'un dispositif médical de la perfusion ; ou
- une information sur le statut d'administration de perfusion de médicament liquide pour un patient n'a pas de prescription correspondante de perfusion de médicament liquide pour ce patient,
dans lequel la deuxième information est reçue à partir du dispositif médical, et
dans lequel le dispositif médical comprend une pompe à perfusion.

2. Système selon la revendication 1, dans lequel la première information comprend au moins un nom de médicament, une dose de médicament, une vitesse d'administration de médicament, le nom du patient et le nom du soignant.

3. Système selon la revendication 2, dans lequel la première information est reçue à partir d'un système d'information hospitalier.

4. Système selon la revendication 1, dans lequel la deuxième information comprend au moins une dose actuelle de médicament fournie à un patient, une vitesse d'administration de médicament du médicament fourni au patient, un nom du médicament fourni au patient, ou un nom du soignant fournissant le médicament au patient.

5. Système selon la revendication 1, dans lequel la première information est reçue par le biais d'un réseau à partir d'un système d'information d'un établissement de soins dans un premier format de message original dudit système d'information de l'établissement de soins, et la deuxième information est reçue par le biais du réseau à partir du dispositif médical dans un second format de message original du dispositif médical, et dans lequel la première information et la deuxième information sont converties dans un format de messagerie interne destiné à être utilisé avec le système de contrôle.

6. Système selon la revendication 1, dans lequel la première information est reçue à partir d'un système de suivi de prescription de médicament et la pompe à perfusion comprend un système d'administration de liquide.

7. Procédé mis en oeuvre par ordinateur pour surveiller l'administration d'un médicament liquide, ledit procédé comprenant :
- la réception de premières informations indiquant des prescriptions de perfusion de médicaments liquides pour patients et des secondes informations indiquant des informations sur le statut d'administration de perfusion de médicaments liquides pour patients ;
- la comparaison des prescriptions de perfusion de médicaments liquides pour patients aux informations sur le statut d'administration de perfusion de médicaments liquides pour patients ; et
- la fourniture d'une notification lorsque la comparaison montre au moins :
- une incohérence entre l'une des prescriptions de la perfusion du médicament liquide selon la première information et l'information sur le statut d'administration de la perfusion du médicament liquide selon des paramètres d'exploitation d'un dispositif médical de la perfusion ; ou
- une information sur le statut d'administration de perfusion de médicament liquide pour un patient n'a pas de prescription correspondante de perfusion de médicament liquide pour ce patient,
dans lequel la deuxième information est reçue à partir d'un dispositif médical, et
dans lequel le dispositif médical comprend une pompe à perfusion.

8. Procédé selon la revendication 7, dans lequel la première information comprend au moins un nom de médicament, une dose de médicament, une vitesse d'administration de médicament, le nom du patient et le nom du soignant.

9. Procédé selon la revendication 8, dans lequel la première information est reçue à partir d'un système d'information hospitalier.

10. Procédé selon la revendication 7, dans lequel la deuxième information comprend au moins une dose actuelle de médicament fournie à un patient, une vitesse d'administration de médicament de médicament du médicament fourni au patient, un nom du médicament fourni au patient, ou un nom du soignant fournissant le médicament au patient.

11. Procédé selon la revendication 7,
dans lequel la notification est fournie par un système de surveillance,
dans lequel la première information est reçue par le biais d'un réseau à partir d'un système d'information d'un établissement de soins dans un premier format de message original dudit système d'information de l'établissement de soins, et la deuxième information est reçue par le biais du réseau à partir du dispositif médical dans un second format de message original du dispositif médical, et
dans lequel la première information et la deuxième information sont converties dans un format de messagerie interne destiné à être utilisé avec le système de contrôle.

12. Procédé selon la revendication 7, dans lequel la première information est reçue à partir d'un système de suivi de prescription de médicament et la pompe à perfusion comprend un système d'administration de liquide.

13. Un moyen de stockage lisible par machine, comprenant des instructions lisibles par machine pour entraîner une unité centrale, après la mise en oeuvre, à mettre en oeuvre un procédé de contrôle d'administration d'un médicament liquide, ledit procédé comprenant :
- la réception de premières informations indiquant les prescriptions de perfusion de médicaments liquides pour patients et des secondes informations indiquant le statut d'administration de perfusion de médicaments liquides pour patients ;
- la comparaison des prescriptions de perfusion de médicaments liquides pour patients aux informations sur le statut d'administration de perfusion de médicaments liquides pour patients ; et
- la fourniture d'une notification lorsque la comparaison montre au moins :
- une incohérence entre l'une des prescriptions de la perfusion du médicament liquide selon la première information et l'information sur le statut d'administration de la perfusion du médicament liquide selon les paramètres d'exploitation d'un dispositif médical de la perfusion ; ou
- une information sur le statut d'administration de perfusion de médicament liquide pour un patient n'a pas de prescription correspondante de perfusion de médicament liquide pour ce patient,
dans lequel la deuxième information est reçue à partir d'un dispositif médical, et
dans lequel le dispositif médical comprend une pompe à perfusion.

14. Moyen de stockage lisible par machine selon la revendication 13, dans lequel la première information comprend au moins un nom de médicament, une dose de médicament, une vitesse d'administration de médicament, le nom du patient et le nom du soignant.

15. Moyen de stockage lisible par machine selon la revendication 13, dans lequel la deuxième information comprend au moins une dose actuelle de médicament fournie à un patient, une vitesse d'administration de médicament du médicament fourni au patient, un nom du médicament fourni au patient, ou un nom du soignant fournissant le médicament au patient.

16. Procédé selon la revendication 7, ledit procédé comprenant en outre :
- la réception d'une indication selon laquelle un patient associé au dispositif médical n'est pas identifié ;
- la fourniture d'une notification indiquant une localisation du dispositif médical dans l'établissement ;
- l'envoi, à un écran d'affichage associé au dispositif médical, d'une requête d'identification du patient associé au dispositif médical ; et
- la réception d'une identification du patient associé au dispositif médical.
